# EUROPÄISCHE PATENTSCHRIFT

(11) **EP 0 730 029 B1**
(45) Veröffentlichungstag und Bekanntmachung des Hinweises auf die Patenterteilung: **03.11.2004**
(21) Anmeldenummer: 96102398.3
(22) Anmeldetag: 17.02.1996
(51) Int. Cl.: C12N 15/31, C07K 14/365, C12N 1/21

(54) **Acarbose-Biosynthesegene aus Actinoplanes sp., Verfahren zu ihrer Isolierung sowie ihrer Verwendung**
Actinoplanes sp. biosynthetic pathway genes for acarbose, isolation and use
Gènes biosynthétiques de l'acarbose obtenus à partir de Actinoplanes sp., procédé d'obtention et utilisation

(30) Priorität: 02.03.1995 DE 19507214
(43) Veröffentlichungstag der Anmeldung: 04.09.1996
(73) Patentinhaber: Bayer HealthCare AG, 51368 Leverkusen (DE)
(72) Erfinder: Crueger, Anneliese, Dr., D-40699 Erkrath (DE); Piepersberg, Wolfgang, Prof. Dr., D-42349 Wuppertal (DE); Distler, Jürgen, Dr., D-42107 Wuppertal (DE); Stratmann, Ansgar, D-42103 Wuppertal (DE)

(56) Entgegenhaltungen:
- DE-A- 2 347 782
- STOCKMANN M ET AL: "GENE PROBES FOR THE DETECTION OF 6-DEOXYHEXOSE METABOLISM IN SECONDARY METABOLITE-PRODUCING STREPTOMYCETES" FEMS MICROBIOLOGY LETTERS, Bd. 90, Nr. 2, 1. Januar 1992, Seiten 185-190, XP002040339
- SIMONET P. ET AL: 'Identification of Frankia strains in nodules by hybridization of polymerase chain reaction products with strain-specific oligonucleotide probes' ARCHIVES OF MICROBIOLOGY Bd. 153, Nr. 3, 1990, Seiten 235 - 240
- URABE H.; OGAWARA H.: 'Cloning, sequencing and expression of serine/threonine kinase-encoding genes from Steptomyces coelicolor A3(2)' GENE Bd. 153, Nr. 1, Februar 1995, Seiten 99 - 104

## Beschreibung

Die Erfindung betrifft Acarbose-Biosynthesegene aus Actinomyceten, vorwiegend aus Actinoplanes sp. SE 50/110 und seiner Mutanten, ein Verfahren zur Isolierung von Acarbose-Biosynthesegenen aus Actinomyceten mittels einer Gensonde, die von hochkonservierten Proteinbereichen bekannter dTDP-Glucose-Dehydratase-Enzyme abgeleitet wurde, für das Auffinden der Gene acbA (kodierend für dTDP-Glucose synthase), acbB (kodierend für dTDP-Glucose dehydratase) und acbC (kodierend für eine Cyclase, z.T. identisch mit AroB, bakteriellen 3-Dehydrochinasäure Synthasen) oder einem oder mehreren Acarbose-Biosynthesegenen aus Actinoplanes sp., sowie die Verwendung der Acarbose-Biosynthesegene.

Gegenstand älterer Patentanmeldungen (z.B. DE 2 064 092, DE 2 209 834) ist die Erkenntnis, daß eine Reihe von Actinomyceten, vor allem die Actinoplanaceen, oligosaccharidartige Inhibitoren von Glycosidhydrolasen vorzugsweise kohlenhydratspaltender Enzyme des Verdauungstraktes bilden. Als potentester Inhibitor dieser Gruppe ist die Verbindung O-4,6-Dideoxy-4-[[1S-(1S, 4R, 5S, 6S)-4,5,6-trihydroxy-3-(hydroxymethyl)-2-cyclohexen-1-yl]-amino]-α-D-glucopyranosyl-(1→4)-O-α-D-glucopyranosyl-(1→4)-D-glucopyranose als Acarbose beschrieben [DE 2 347 782].

Acarbose wird bei der Bekämpfung des Diabetes mellitus in der Humanmedizin eingesetzt. Die Bildung des Sekundärmetaboliten Acarbose erfolgt durch Actinoplanes sp. SE 50 (CBS-Nr. 961.70) und durch eine natürliche Variante dieses Stammes, SE 50/110 (CBS 614.71) [DE 22 09 834], sowie durch deren Selektanten und Mutanten. Das ursprüngliche Isolat stammt aus Kenia, bei Ruiru. In den genannten Patentanmeldungen, z.B in den Beispielen 1 bis 4 der genannten deutschen Patentanmeldung P 20 09 834, wird die Gewinnung eines derartigen Saccharase-Inhibitors beschrieben.

Aus der Struktur der Acarbose war zu vermuten, daß der Deoxyglucose-Teil des Acarbose-Moleküls entsprechend der Biosynthese von 6-Deoxyzuckerresten verschiedener Antibiotika gebildet wird (z. B. Aminoglykoside, wie Streptomycin, Kasugamycin; Makrolide, wie Erythromycin, Tylosin; Polyenen, wie Amphotericin A, B, Nystatin; Anthrazycline, wie Daunorubicin; Glykopeptide, wie Vancomycin).

Mittels Gentechnologie lassen sich bestimmte Gene direkt aus dem Genom isolieren, wobei z.B. Gensonden eingesetzt werden, die spezifisch an die gewünschte DNA-Sequenz binden. Damit kann das zu isolierende Gen aus einer großen Anzahl unbekannter Sequenzen "gefischt" werden.

Weiterhin ist bei Actinomyceten - vor allem Streptomyceten - bekannt, daß bei den bisher untersuchten Sekundärmetabolit-Produzenten die Biosynthesegene auf dem Chromosom, seltener auf einem Plasmid, in einem "Cluster" nebeneinander angeordnet sind [Martin, J.F., J. Ind. Microbiol 9, 73-90 (1992), Chater, K.F., Ciba Found. Symp., 171 (Second Metabolites: Their Function and Evolution) 144-62 (1992)]. Damit lassen sich durch das "Fischen" mit Gensonden benachbarte, bisher unbekannte Biosynthesegene isolieren, deren Bedeutung für die gewünschte Biosynthese dann aufgeklärt werden kann.

Weiterhin ist dem Fachmann aus DE-A-2347782 bekannt, das Actinoplanaceen, speziell *Actinoplanes* SE 50/110, Acarbose synthetisieren können. Es ist allerdings nicht offenbart, auf welchem Wege man bei diesem Organismus die dafür notwendigen Biosynthesegene isolieren kann.

Aus Stockmann et al (1992, FEMS Microbiology Letters, 90(2), 185-190) ist eine Methode bekannt, mit deren Hilfe 6-Deoxyhexose-Biosynthesegene in *Actinomyceten* detektiert werden können. Diese Methode führt aber mitunter nicht zum erwarteten Erfolg. In Fällen, in denen die Methode versagt, ist der Fachmann weiterhin nicht in der Lage die gewünschten Biosynthesegene aus Actinomyceten zu erhalten.

Aus Urabe et al. (1995, Gene, 153(1), 99-104) ist bekannt, dass Primer aus hochkonservierten Bereichen von Ser/Thr Proteinkinasen zur Isolierung selbiger Enzyme aus *Streptomyces coelicolor* verwendet werden können. Es wird allerdings nicht offenbart, welche speziellen Primer-Sequenzen verwendet werden müssten, um die beschriebene Technik auf andere Spezies anzuwenden.

Entsprechende Anwendung molekularbiologischer Techniken war bei Actinoplanaceen bisher noch nicht gelungen und war bei dieser genetisch nicht charakterisierten Organismengruppe auch nicht zu erwarten.

Überraschend wurde nun gefunden, daß sich eine Gensonde, die von hochkonservierten Proteinbereichen bekannter dTDP-Glucose-Dehydratase-Enzyme abgeleitet wurde, in Actinoplanes sp. für das Auffinden der Gene acbA (kodierend für dTDP-Glucose Synthase) und acbB (kodierend für dTDP-Glucose Dehydratase) eignet. Überraschend ist weiterhin, daß als weiteres Gen das dem acbB Gen benachbarte acbC gefunden wurde, das für eine Cyclase kodiert (z.T. identisch mit AroB, bakteriellen 3-Dehydrochinasäure Synthasen) und somit an der Biosynthese des ungesättigten Cyclitols (Valienamine) beteiligt ist.

Die vorliegende Erfindung betrifft:

Die vollständige DNA-Sequenz von acbB sowie die DNA-Teilsequenzen von acbA und acbC.

Die vollständige Aminosäuresequenz von acbB sowie die Aminosäureteilsequenzen von acbA und acbC.

Ein Verfahren zur Isolierung von Sekundärmetabolit-Biosynthesegenen aus Actinomyceten, vor allem aus Actinoplanes, dadurch gekennzeichnet, daß eine Gensonde, die von hochkonservierten Proteinbereichen bekannter dTDP-Glucose-Dehydratase-Enzyme abgeleitet wurde, für das Auffinden der Gene acbA (kodierend für dTDP-Glucose Synthase), acbB (kodierend für dTDP-Glucose Dehydratase) und acbC (kodierend für eine Cyclase, z.T. identisch mit AroB, bakteriellen 3-Dehydrochinasäure Synthasen) oder einem oder mehreren Acarbose-Biosynthesegenen aus Actinoplanes sp. verwendet wird.

Ein Verfahren zur Isolierung von Biosynthesegenen von Acarbose-verwandten Naturstoffen in Actinomyceten (z.B. für Validamycin, Oligostatine (Trestatin), Adiposine).

Steigerung der Acarbose-Syntheseleistung in Actinoplanes durch erhöhte Gendosis oder effektivere Promotoren oder durch gentechnische Veränderung der Regulation (z.B. der Regulatorproteinexpression oder der Regulator-Erkennungsstellen).

Die Steigerung der Acarbose-Syntheseleistung in Actinoplanes durch Protein Engineering bei den die Acarbose-Synthese limitierenden Biosyntheseschritten ("Bottleneck"-Enzyme) oder zur Vermeidung von Nebenkomponentenbildung.

Eine Eingrenzung des Produktspektrums in Actinoplanes auf das gewünschte Hauptprodukt durch Ausschalten unerwünschter Biosynthesewege zu Nebenkomponenten oder unerwünschter enzymatischer Abbaureaktionen.
Die Veränderung der Regulation und damit der Nährstoffbedürfnisse in Hinblick auf eine verbesserte Acarbose-Produktivität in Actinoplanes.

Eine Expression in heterologen Wirtsstämmen (z.B. in Streptomyces lividans sowie in anderen Streptomyceten, in schnell wachsenden Bakterien wie E. coli, Bacillus subtilis oder Corynebacterium glutamicum, oder in Hefen), um
- durch verbesserte Raum-Zeit-Ausbeuten eine Produktionssteigerung zu erzielen,
- ein vereinfachtes Verfahren für die Aufreinigung zu entwickeln,
- eine gezielte Einschränkung des Produktspektrums zu erzielen.

Die Verwendung der Acarbose-Biosynthesegene für die in vitro-Synthese von Acarbose oder Verbindungen dieser Stoffklasse, ausgehend von synthetisch oder mikrobiell hergestellten Vorstufen.

Die Erfindung wird im folgenden detailliert beschrieben.

Alle gentechnologischen Methoden wurden, wenn nicht anders angegeben, wie in J. Sambrook et al. (Molecular Cloning; A laboratory manual; 2nd edition, 1989; Cold Spring Harbor Laboratory Press, N.Y., USA) durchgeführt.

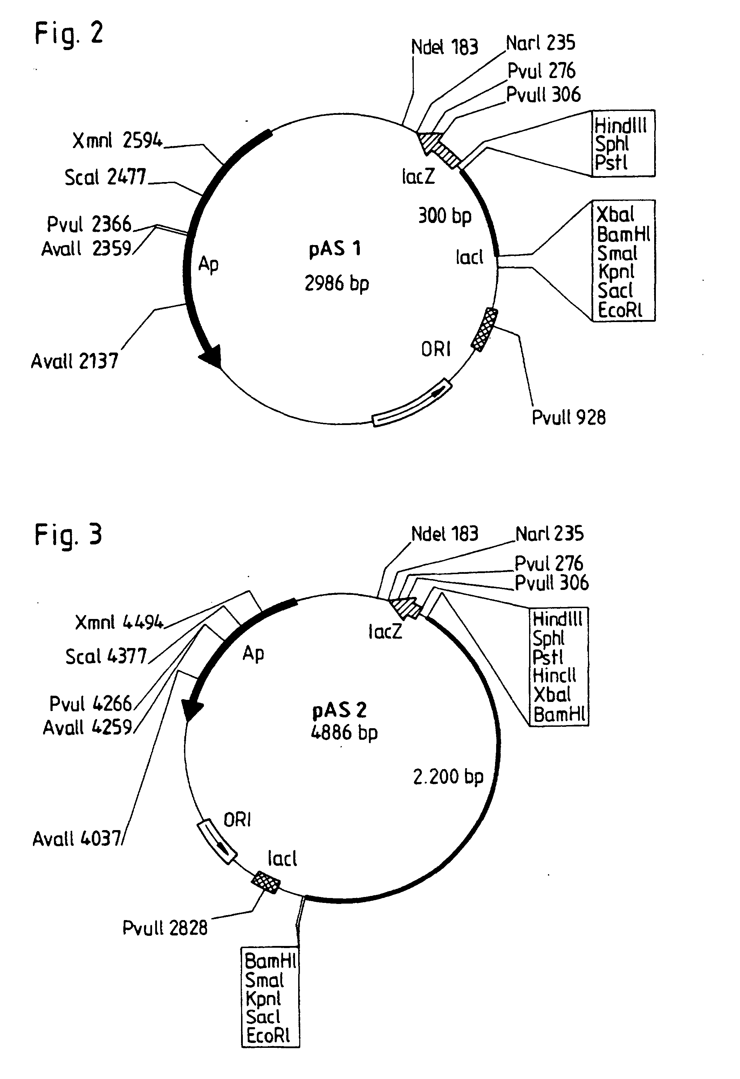

Die für das Screening verwendete Gensonde wurde mittels PCR (polymerase chain reaction) mit Oligonukleotid-Primern (s. Tabelle 1), die von hochkonservierten Proteinbereichen bekannter dTDP-Glukose-Dehydratase-Enzyme abgeleitet wurden, aus Actinoplanes sp. SE50/110 gewonnen. Das amplifizierte Actinoplanes sp. SE50/1210 DNA-Fragment wurde in pUC18 kloniert und in E. coli DH5α (Gibco BRL, Eggenstein, Deutschland) transformiert. Das resultierende Plasmid (pAS1, s. Abbildung 2) wurde aus E. coli mit Hilfe der "Boiling-Method" oder durch alkalische Lyse isoliert (Sambrook et al., 1989).

Das Plasmid pAS1 aus E. coli DH5α wurde mit den Restriktionsenzymen EcoRI und HindIII hydrolysiert. Das 0,3 kb EcoRI/HindIII-Fragment wurde isoliert und durch sogenannte "Nick-translation" mit ³²P-markierten Desoxynukleotiden markiert. Dieses radioaktiv markierte Fragment wurde als Gensonde für die Isolierung von Acarbose-Biosynthese-Genen eingesetzt und wird im folgenden als acb-Sonde bezeichnet.

Acarbosebiosynthese-Gene wurden wie folgt isoliert. Chromosomale DNA von Actinoplanes sp. wurde mit verschiedenen Restriktionsenzymen (SstI, BglII und BamHI) gespalten, Gel-chromatographisch getrennt und durch "Southern"-Hybridisierung mit der acb-Sonde nach homologen Genen untersucht. Mit der Gensonde hybridisierende DNA-Restriktionsfragmente haben folgende Größen: 10 kb (SstI), 9-10 kb (BglII) und 2,2 kb (BamHI). Die unterschiedlichen mit der acb-Sonde hybridisierenden DNA-Fragmente wurden aus dem Gel eluiert, in den Vektor pUC 18 ligiert und in E. coli DH5α kloniert.

Bevorzugt wurde das 2,2 kb BamHI-DNA-Fragment kloniert und sequenziert. E. coli DH5α-Klone, die mit der acb-Sonde hybridisierende Actinoplanes sp. DNA enthalten, wurden durch Pool-Hybridisierung identifiziert (s. Punkt 7). Aus den positiven Klonen wurde die Plasmid-DNA isoliert und durch "Southern"-Hybridisierung das Ergebnis der Pool-Hybridisierung verifiziert. Das erhaltene Plasmid (s. Abbildung 3) wurde einer Spaltung mit verschiedenen Restriktionsendonukleasen unterzogen, und die entstehenden DNA-Fragmente in pUC18 in E. coli DH5α subkloniert, bzw. religiert und sequenziert.

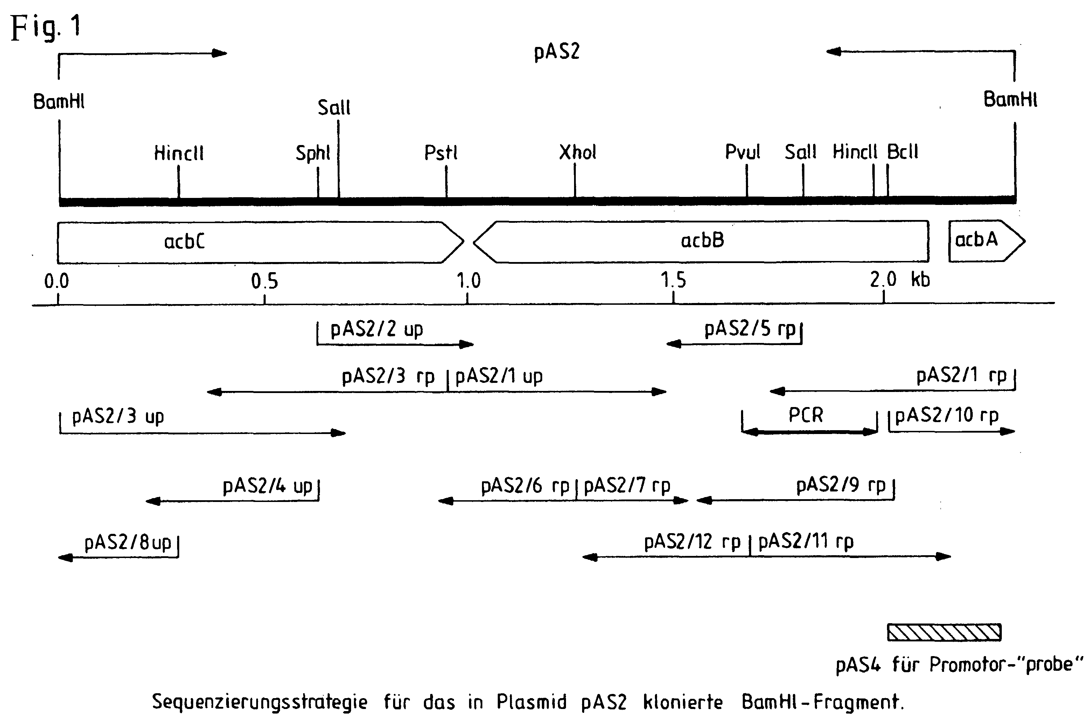

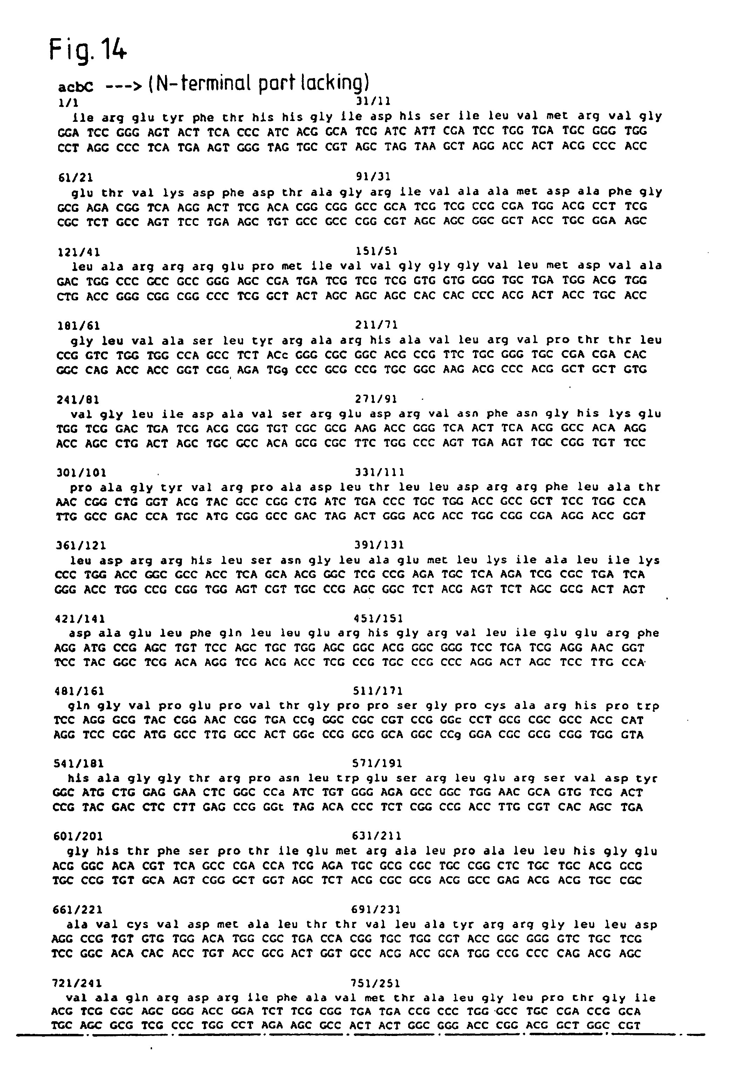

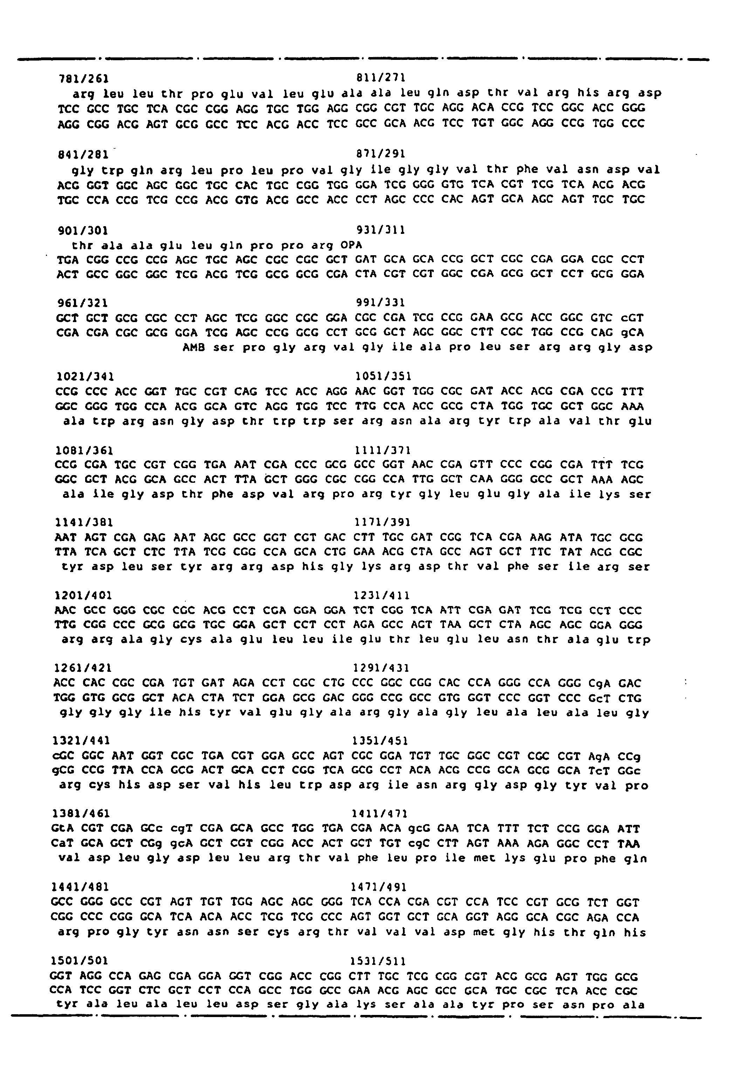

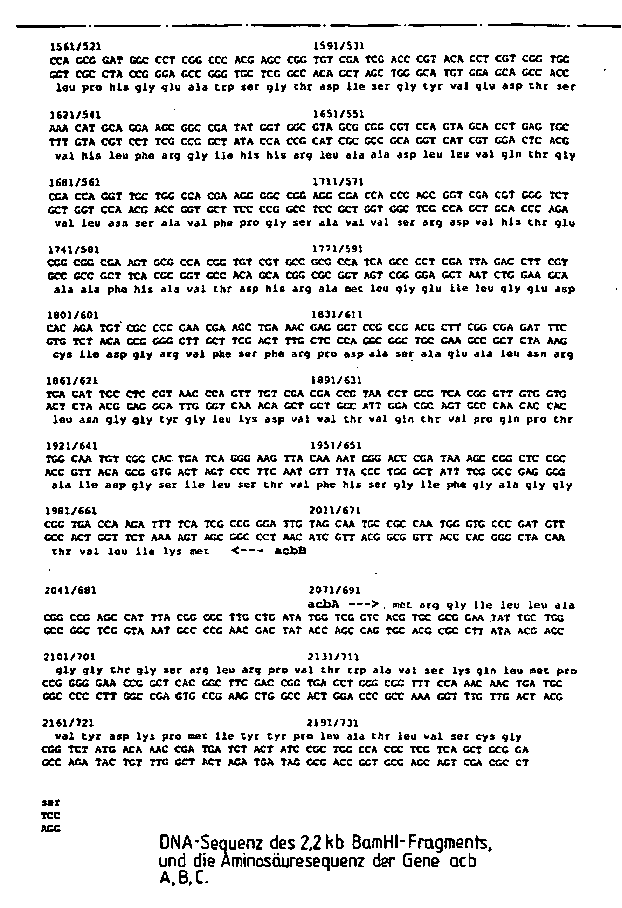

Für die Bestimmung der DNA-Sequenz des 2,2 kb BamHI-Fragments von Actinoplanes sp. wurden folgende Plasmide konstruiert (s. Punkt 7), und die Sequenz der Insert-DNA analysiert: (s. Abbildung 1, 14)

| | | |
|---|---|---|
| pAS 2/1 = | 1,3 kb | PstI / BamHI Fragment aus pAS2 (Abb. 4) |
| pAS 2/2 = | 1,6 kb | SphI / BamHI Fragment aus pAS2 (Abb. 5) |
| pAS 2/3 = | 0,9 kb | PstI Fragment aus pAS2 (Abb.6) |
| pAS 2/4 = | 0,6 kb | SphI Fragment aus pAS2 (Abb.7) |
| pAS 2/5 = | 0,8 kb | HincII / HindIII Fragment aus pAS2/1 (Abb.8) |
| pAS 2/6 = | 0,65 kb | XhoI / SalI Fragment aus pAS2 (Abb.9) |
| pAS 2/7 = | 0,5 kb | XhoI / SalI Fragment aus pAS2 (Abb. 10) |
| pAS 2/8 = | 0,28 kb | HincII Fragment aus pAS2/3 (Abb. 11) |
| pAS 2/9 = | 0,7 kb | XhoI / BclI Fragment aus pAS2/1 (Abb. 12) |
| pAS 2/10 = | 0,3 kb | SstI / BclI Fragment aus pAS2 (Abb. 13) |

Für die DNA-Sequenzierung wurde die Methode von F. Sanger et al. (1977) oder ein davon abgeleitetes Verfahren angewandt. Es wurde mit dem Autoread Sequencing Kit (Pharmacia, Freiburg, Deutschland) in Verbindung mit dem Automated Laser Fluoreszens (ALF) DNA-Sequenzierungsgerät (Pharmacia, Freiburg, Deutschland) gearbeitet. Geeignete Fluoreszein-markierte pUC reverse sequencing und sequencing Primer wurden käuflich erworben (Pharmacia, Freiburg, Deutschland; s. Tabelle 1).
Primer für die PCR: 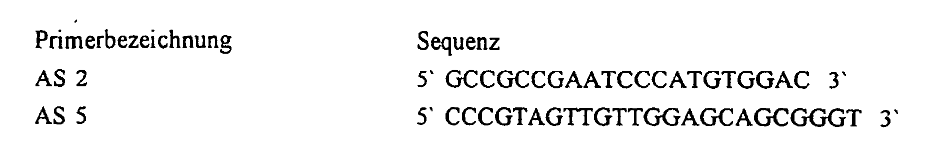
Primer für die Sequenzreaktion:

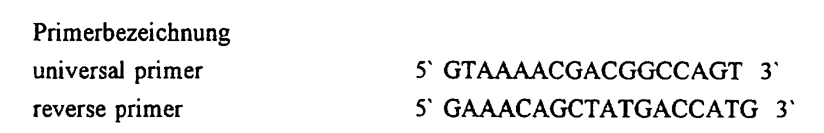

### Beispiele

### Beispiel 1

### Anzucht der E. coli Stämme, Präparation der Plasmid-DNA und Isolierung

E. coli DH5α wurde in LB-Medium bei 37°C bebrütet. Plasmidtragende Bakterien wurden unter Selektionsdruck (Ampicillin, 100 µg/ml) gehalten. Die Kultivierung erfolgte auf einem Rundschüttler bei 270 rpm. Als Übernachtkultur (ÜK) wurden Ansätze bezeichnet, die wenigstens 16 h bebrütet wurden.

Für die Präparation von Plasmid-DNA wurden die Zellen aus 1,5 ml einer unter Selektionsdruck bebrüteten ÜK eingesetzt. Die Isolierung der Plasmide erfolgte nach der Methode der alkalischen SDS-Lyse (Birnboim & Doly 1979).

Zur gezielten Hydrolyse von Vektor-DNA wurden ausschließlich Restriktionsendonukleasen nach Vorschrift des Herstellers (Gibco BRL, Eggenstein, Deutschland) eingesetzt. Zur Restriktion von 10 µg Plasmid-DNA wurden 5 U der jeweiligen Restriktionsendonuklease eingesetzt und 2 h bei 37°C inkubiert. Um eine vollständige Hydrolyse zu gewährleisten wurde die gleiche Menge Restriktionsendonuklease ein zweites Mal zugegeben und erneut mindestens 1 h inkubiert.

Die gespaltene DNA wurde, je nach Größe der DNA-Fragmente, auf 0,5 - 1,2 %igen horizontalen Agarosegelen elektrophoretisch getrennt. Zur Elution wurde das Gelstück, welches das DNA-Fragment enthielt, mit einem sterilen Skalpell ausgeschnitten und gewogen. Die Elution der DNA-Fragmente aus der Agarose erfolgte nach Vorschrift mit dem JETsorb-Kit (Genomed, Bad Oeynhausen, Deutschland).

### Beispiel 2

### Anzucht von Actinoplanes sp. Präparation, Spaltung der chromosomalen DNA und gelelektrophoretische Trennung

Actinoplanes sp. SE50/110 wurde bei 30°C in TSB-Medium auf einem Rundschüttler für 3 d bebrütet. Die Vorkultur (5 ml) erfolgte bei 240 rpm in Kulturröhrchen, die Hauptkultur (50 ml) in 500 ml Schikanekolben bei 100 rpm. Die Zellen wurden nach der Kultivierung durch Zentrifugation sedimentiert und zweimal in TE-Puffer gewaschen.

Die Präparation der Gesamt-DNA erfolgt mit 1,5 - 2 mg Zellen (Frischgewicht) nach der Methode der Phenol/Chloroform-Extraktion (D.A. Hopwood et. al. 1985).

Die Hydrolyse von 20 µg chromosomaler DNA wurde mit 10 U des entsprechenden Restriktionsenzyms (Gibco BRL, Eggenstein, Deutschland) für 2 h bei 37°C in dem zugehörigen Puffer durchgeführt. Um eine vollständige Hydrolyse zu gewährleisten, wurde die gleiche Menge Restriktionsendonuklease ein zweites Mal zugegeben und erneut für mindestens 1 h inkubiert.

Die gespaltene DNA wurde auf 0,7 %igen horizontalen Agarosegelen elektrophoretisch getrennt.

Die Elution von DNA-Fragmenten erfolgte wiederum mit dem JETsorb-Kit (s. Punkt 1).

### Beispiel 3

### Herstellung der acb-Gensonde

Das nach Punkt 1 präparierte Fragment aus pAS1 wurde mit dem "Nick Translation System" des Herstellers Gibco BRL, Eggenstein, Deutschland nach dessen Angaben radioaktiv markiert. Hierbei wurden 0,5 - 1,0 µg DNA-Fragment eingesetzt. Es wurde [α³²P]dCTP eingesetzt (3 000 Ci/mmol; Amersham, Braunschweig, Deutschland). Anschließend wurde der Ansatz 10 Minuten gekocht (Denaturierung) und sofort zu der Hybridisierungslösung gegeben (s. Punkt 4).

### Beispiel 4

### DNA-Transfer auf Membranen. DNA-Hybridisierung (Southern-Hybridisierung) und Autoradiographie

Die Übertragung von DNA-Fragmenten aus Agarosegelen auf Membranen erfolgt nach der Methode des "Southern-Transfer" (Southern, E.M., 1975). Die nach Punkt 2 erhaltenen Agarosegele wurden 20 Minuten in 0,25 M HCl geschwenkt. Die Gele wurden auf 3 Lagen saugfähigen Whatman-Papier 3MM (Whatman, Maidstone, England) gelegt und eine Hybond™-N+Membran (Amersham, Braunschweig, Deutschland) luftblasenfrei aufgelegt. Darauf wurden mehrere Schichten saugfähiges Papier gelegt. Auf den Filterstapel wurde ein ca. 1 kg schweres Gewicht gestellt. Der DNA-Transfer erfolgt durch Durchsaugen von 0,4 m NaOH. Nach mindestens 12 h Transferzeit wurden die Nylonfilter mit 2xSSC für 5 Minuten gespült und an der Luft getrocknet.

Die Nylon-Filter wurden dann mindestens 2 h bei 68°C in 50 - 100 ml Prähybridisierungslösung im Wasserbad geschüttelt. Dabei wurde die Lösung mindestens zweimal gewechselt. Die Hybridisierung fand im Hybridisierungschrank für mindestens 12 h statt. Es wurden 15 ml Hybridisierungslösung, welche die acb-Sonde enthielt (s. Punkt 3), eingesetzt.

Die Nylon-Filter wurden anschließend für jeweils 15 Minuten mit 6x Postwash und 1x Postwash gewaschen. Die Nylon-Filter wurden dann im noch feuchten Zustand mit Frischhaltefolie abgedeckt. Die Autoradiographie erfolgt mit Hyperfilm-MP (Amersham, Braunschweig, Deutschland) in einer lichtdichten Kassette mit Verstärkerfolien bei -80°C für mindestens 16 h.

### Beispiel 5

### Isolierung und Klonierung von BamHI-Fragmenten aus der Gesamt-DNA von Actinoplanes sp.

Chromosomale DNA aus Actinoplanes sp. SE50/110 wurde mit BamHI vollständig hydrolysiert, durch Agarose-Gelelektrophorese getrennt und die DNA-Fragmente der Länge 1,5 - 3 kb aus der Agarose eluiert. Das Vektorplasmid pUC18 wurde aus E. coli DH5α präpariert, mit BamHI hydrolysiert und mit alkalischer Phosphatase (Boehringer, Mannheim, Deutschland) nach Vorschrift des Herstellers behandelt. Die Ligation fand in einem Volumen von 20 µl statt, wobei das Verhältnis von Fragment zu Vektor 3:1 betrug mit 0,01 - 0,1 µg DNA im Ansatz. Es wurde 1 U der T4-DNA-Ligase mit dem entsprechendem Puffer (Gibco BRL, Eggenstein, Deutschland) eingesetzt. Transformationskompetente Zellen von E. coli DH5α wurden mit vollständigen Ligationsansätzen transformiert (nach Hanahan 1983). Ampicillin-resistente Transformanten wurden auf LB-Amp Selektivplatten (100 µg/ml) übertragen.

### Beispiel 6

### Identifizierung von Klonen, welche das dTDP-D-Glucose-Synthasegen enthalten

Ampicillin-resistente Transformanten wurden auf das Vorhandensein des dTDP-D-Glucose-Synthasegens untersucht. Jeweils zehn dieser Klone wurden auf einer Selektivplatte ausgestrichen, über Nacht bebrütet und mit 3 ml LB-Medium von der Platte gewaschen. Es wurde dann aus 20 solcher Zehner-Pools die Plasmid-DNA isoliert (nach Birnboim & Doly, 1979). Um die klonierten BamHI-Fragmente aus dem Polylinker zu entfernen, wurden die 20 verschiedenen Plasmidpräparationen mit den Restriktionsendonukleasen EcoRI und HindIII hydrolysiert. Die Restriktionsansätze wurden dann auf einem 0,8 % Agarosegel elektrophoretisch getrennt, und die DNA mittels Southern-Transfer aus dem Agarosegel auf einen Nylon-Filter übertragen (s. Punkt 4). Die Hybridisierung erfolgte wiederum mit der acb-Sonde (s. Punkt 4). Einer der Pools reagierte positiv mit der acb-Sonde und wurde in die zehn Einzelklone aufgetrennt. Deren Plasmide wurden ebenfalls isoliert und der oben beschriebenen Prozedur unterworfen. Das hybridisierende Plasmid wurde mit pAS2 bezeichnet. Es enthält ein 2,2 kb BamHI-Fragment.

### Beispiel 7

### Subklonierung des Plasmids pAS2

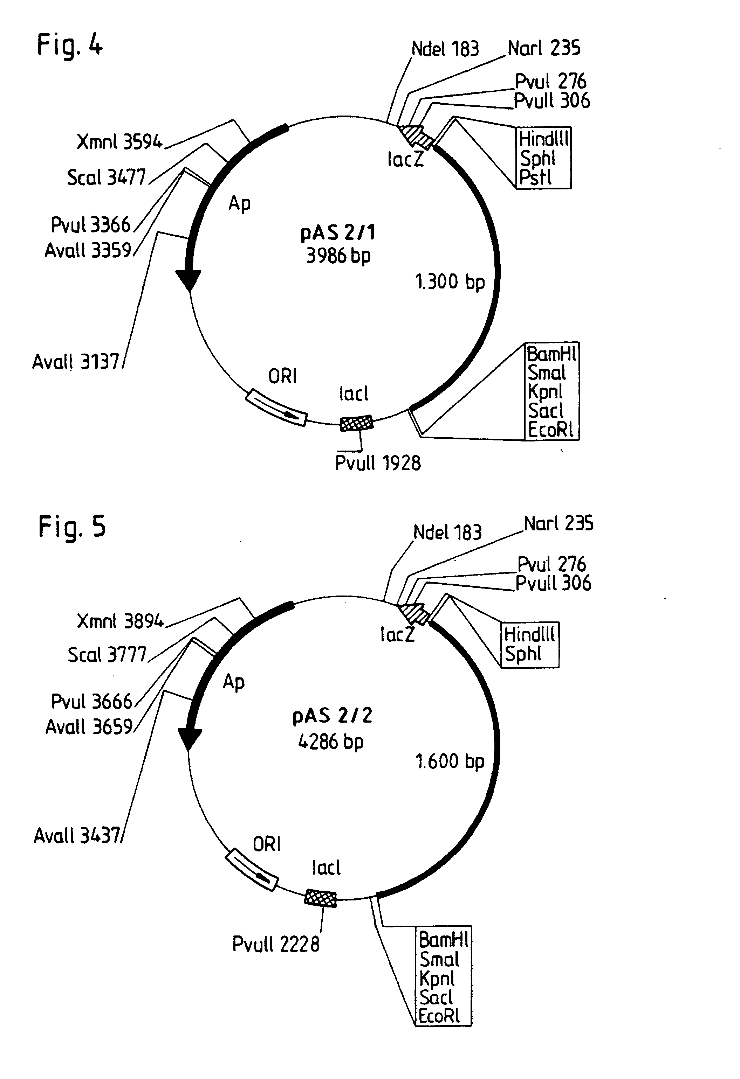

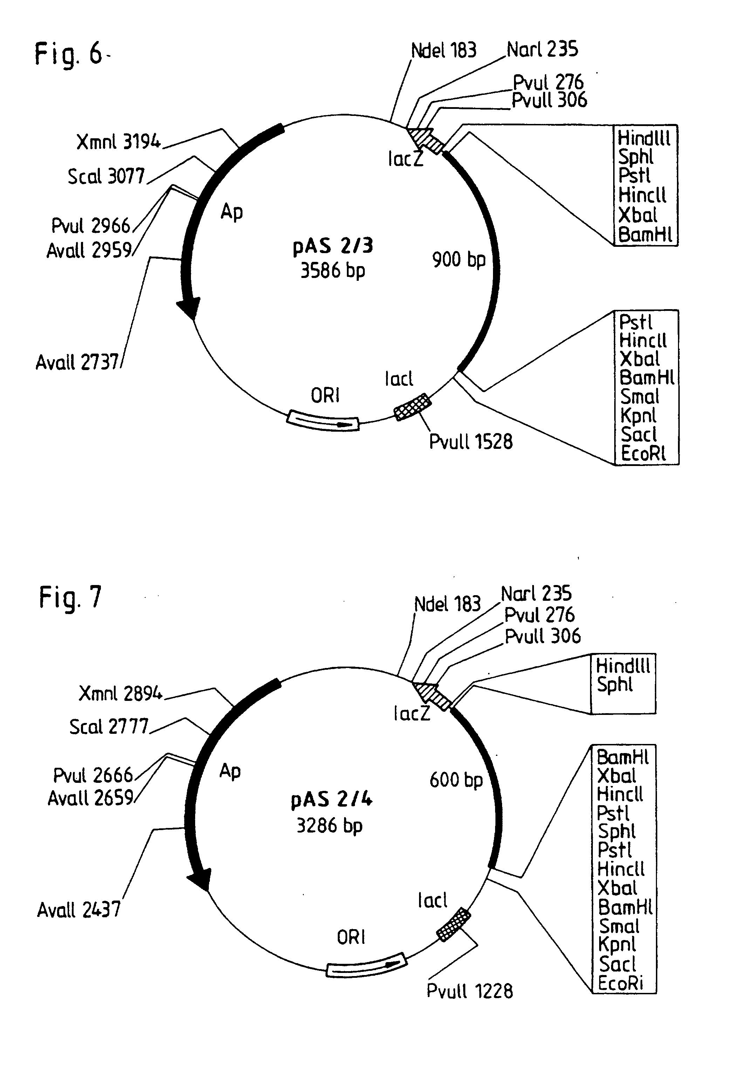

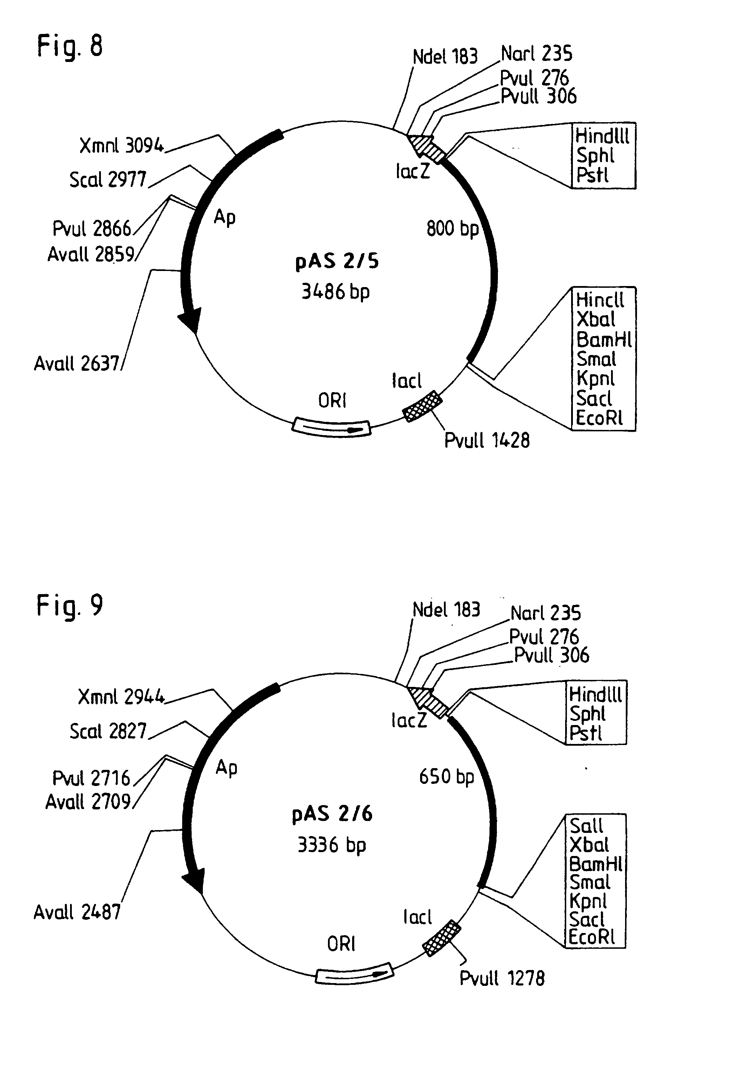

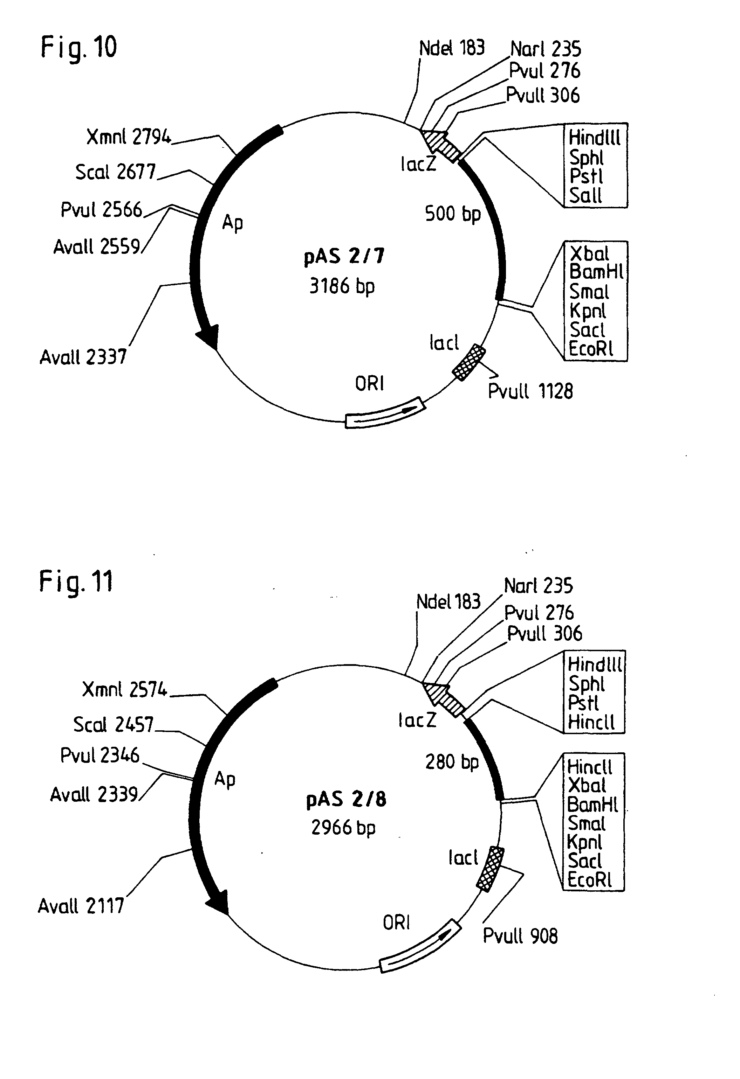

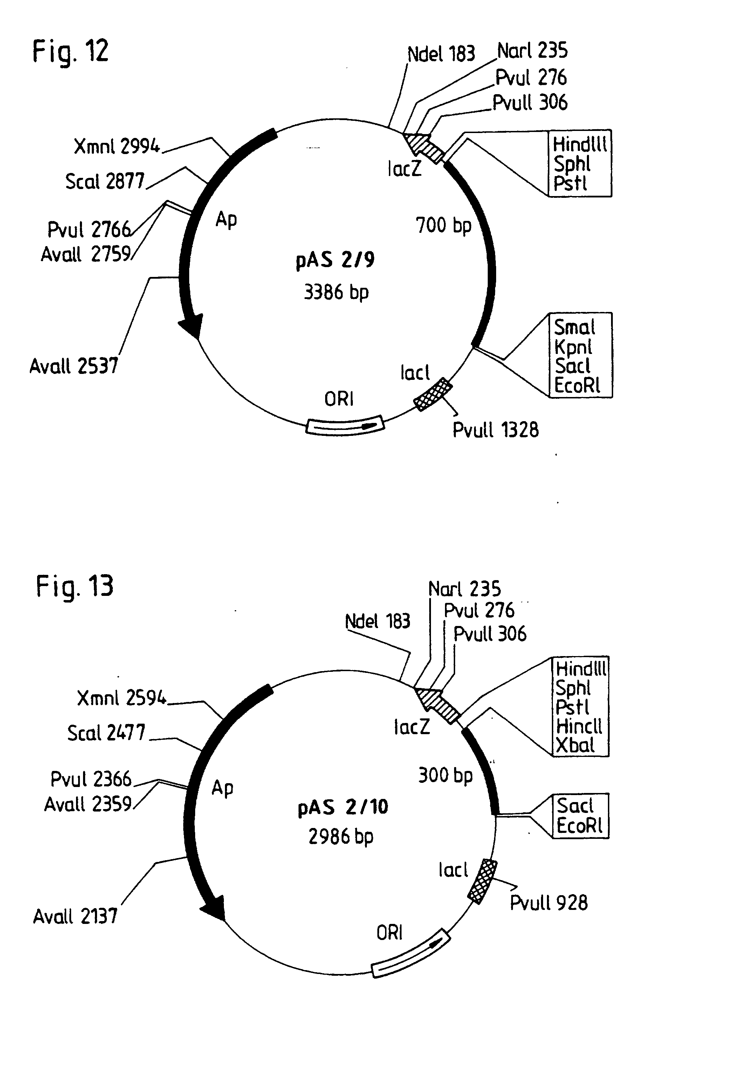

Ausgehend von dem Plasmid pAS2 wurden mehrere Subklone erstellt, um die Sequenz der Doppelstrang-DNA aufzuklären (Abbildung 1; Abbildung 4-13).

### pAS2/1 und pAS2/3)

Das Plasmid pAS2 wurde mit PstI hydrolysiert. Der Restriktionsansatz wurde auf einem 1 %igem Agarosegel getrennt. Das durch die Hydrolyse entstandene 0,9 kb PstI-Fragment und die Plasmidbande mit dem restlichen 1,3 kb PstI/BamHI-Fragment wurden aus dem Agarosegel eluiert (JETsorb-Kit; Genomed, Bad Oeynhausen, Deutschland). Das Plasmid mit dem 1,3 kb PstI/BamHI-Fragment wurde zum Subklon pAS2/1 religiert. Das 0,9 kb PstI-Fragment wurde in den Vektor pUC18 (hydrolysiert mit PstI) kloniert und es entstand der Subklon pAS2/3.

### pAS2/2 und pAS2/4)

Das Plasmid pAS2 wurde mit SphI hydrolysiert. Das Plasmid mit dem 1,6 kb SphI/BamHI-Fragment wurde zum Subklon pAS2/2 religiert. Das 0,6 kb SphI-Fragment wurde in pUC18 (hydrolysiert mit SphI) kloniert und es entstand der Subklon pAS2/4.

### pAS2/5)

Das Plasmid pAS2/1 wurde mit HincII/HindIII hydrolysiert. Das dadurch entstandene 0,8 kb Fragment wurde in pUC18 (hydrolysiert mit HindIII/HincII) kloniert und es entstand der Subklon pAS2/5.

### pAS2/6)

Das Plasmid pAS2 wurde mit XhoI/SalI hydrolysiert. Das dadurch entstandene 0,65 kb Fragment wurde in pUC18 (hydrolysiert mit SalI) kloniert und es entstand der Subklon pAS2/6.

### pAS2/7)

Das Plasmid pAS2 wurde mit XhoI/SalI hydrolysiert. Das dadurch entstandene 0,5 kb Fragment wurde in pUC18 (hydrolysiert mit SalI) kloniert und es entstand der Subklon pAS2/7.

### pAS2/8)

Das Plasmid pAS2/3 wurde mit HincII hydrolysiert. Das dadurch entstandene 0,3 kb Fragment wurde in pUC18 (hydrolysiert mit HincII) kloniert und es entstand der Subklon pAS2/8.

### pAS2/9)

Das Plasmid pAS2/1 wurde mit XhoI/BclI hydrolysiert. Das dadurch entstandene 0,7 kb Fragment wurde in pUC18 (hydrolysiert mit SalI/BamHI) kloniert und es entstand der Subklon pAS2/9.

### pAS2/10

Das Plasmid pAS2 wurde mit SstI/BclI hydrolysiert. Das dadurch entstandene 0,3 kb Fragment wurde in pUC18 (hydrolyisert mit SstI/BamHI) kloniert und es entstand der Subklon pAS2/10.

### Beispiel 8

### DNA-Sequenzierung des 2,2 kb BamHI-Fragments von Actinoplanes sp.

Es wurden die unter Punkt 7 beschriebenen Plasmide sequenziert. In die Sequenzierungsreaktion wurden 6 - 8 µg Plasmid-DNA aus einer Präparation (s. Punkt 1) eingesetzt. Die Sequenzierreaktion wurde mit dem Auto-Read-Sequenzing-Kit (Pharmacia, Freiburg, Deutschland) durchgeführt. Hierbei kam das Standardprotokoll zur Sequenzierung von dsDNA zur Anwendung. Um die Auswertung der Nukleotidsequenz mit dem A.L.F (automatisierter Laser Fluoresens-(DNA)-Sequenzierer) zu ermöglichen, wurden als Startermoleküle für die Sequenzreaktion die Fluorescein-markierten universellen und reversen Sequenzier-Primer verwendet (s. Tabelle 1). Zur Herstellung des Gels wurden 8 ml Hydro Link Long Ranger (Serva, Heidelberg, Deutschland), 33,6 g Harnstoff, 8 ml 10x TBE-Puffer, ad 80 ml mit H₂O vermischt, sterilfiltriert und 1 Minute entgast. Die Polymerisation wurde durch Zugabe von 350 µl 10 % (w/v) Ammoniumpersulfat und 40 µl N,N,N',N'-Tetramethylethylendiamin eingeleitet. Die Lösung wurde in eine Gelform (50x50x0,05 cm) gegossen. Die Elektrophorese erfolgte bei 38 W und einer konstanten Temperatur von 45°C. Als Laufpuffer diente 1x TBE-Puffer. Die Verarbeitung der gemessenen Fluoreszenz zu einer DNA-Sequenz erfolgte über einen angeschlossenen Computer (Compaq 386/20e), der auch zur Steuerung der Elektrophoreseeinheit diente (Programm A.L.F. Manger 2,5; Pharmacia).

### Puffer und Lösungen:

### Medien für die Bakterienanzucht

| LB-Medium: | |
|---|---|
| Trypton | 10 g |
| NaCl | 10 g |
| Hefeextrakt | 5 g |
| H₂O | ad 1 000 ml |

Es wurde mit 4 M NaOH ein pH-Wert von 7,5 eingestellt

| TSB-Medium: | |
|---|---|
| Tryptone-Soya Broth (Oxoid) | 30 g |
| H₂O | ad 1 000 ml |

| TE-Puffer (pH 8,0) | |
|---|---|
| Tris-HCl | 10 mM |
| Na₂-EDTA | 1 mM |

### Standardpräparation von Plasmid-DNA

| (modif. nach Birnboim & Doly 1979) | |
|---|---|
| Mix I: | 50 mM Glukose |
| | 50 mM Tris-HCl (pH 8,0) |
| | 10 mM EDTA (pH 8,0) |
| | 5 mg/ml Lysozym |
| | |
| Mix II: | 200 mM NaOH |
| | 1 % (w/v) SDS (Natriumdodecylsulfat) |
| | |
| Mix III: | 3 M Kaliumacetat |
| | 1,8 M Formiat |

### DNA-DNA-Hybridisierung

| 20 x SSC: |
|---|
| 3 M NaCl |
| 0,3 M Na-Citrat |
| pH 7,2 |

### Prähybridisierungslösung:

| 6 x SSC: |
|---|
| 0,01 M Natriumphosphatpuffer pH 6,8 |
| 1 mM EDTA |
| 0,5 % SDS |
| 0,1 % Magermilchpulver |

### Hybridisierungslösung:

In 15 ml Prähybridisierungslösung wird die acb-Sonde nach der Markierungsreaktion gegeben.

| 6x Postwash: |
|---|
| 6x SSC |
| 0,5 % SDS |

### DNA-Sequenzierung:

| TBE-Puffer (pH 8,0): |
|---|
| 1 M Tris-Base |
| 0,83 M Borsäure |
| 10 mM EDTA |

### Literatur

1. Birnboim H.C. & Doly J. (1979)
   A rapid alkaline extraction procedure for screening recombinant plasmid DNA
   Nucleic Acids Res.; 7, 1513 - 1523
2. Hanahan D. (1983)
   Studies on transformation of Escherichia coli with plasmids
   J. Mol. Biol.: 166, 557 - 580
3. Hopwood D.A. et. al, (1985)
   Genetic manipulation of Streptomyces;
   A laboratory manual; The John Innes Foundation, Norwich, England
4. Sanger F.; Nicklen S.; Coulson A.R. (1977)
   DNA sequencing with chain-terminating inhibitors
   Proc. Natl. Acad. Sci. USA, 74, 5463 - 5467
5. Southern E.M., (1975)
   Detection of specific sequences among DNA Fragments separated by gel electrophoresis
   J. Mol. Biol., 98, 503 - 521

## Patentansprüche

1. DNA-Molekül gemäß SEQ ID 01.

2. Fragmente von DNA-Molekülen gemäß Anspruch 1, enthaltend
(i) die vollständige acbB-DNA-Sequenz, kodierend für die dTDP-Glucose Dehydratase, und/oder
(ii) eine Teilsequenz des Gens acbA, kodierend für dTDP-Glucose Synthase mit einer Sequenz gemäß Position 2081 bis 2223 in SEQ ID NO: 1, und/oder
(iii) eine Teilsequenz des Gens acbC, kodierend für eine Cyclase mit einer Sequenz gemäß Position 1 bis 929 in SEQ ID NO: 1.

3. Vektoren enthaltend eine DNA gemäß den Ansprüchen 1 und 2.

4. Mikroorganismen transformiert mit dem Vektor gemäß Anspruch 3.

5. Polypeptide kodiert durch DNA Moleküle gemäß Anspruch 1 und 2.

6. Verfahren zur Isolierung von einem oder mehreren Acarbose-Biosynthesegenen aus Actinoplanes, **dadurch gekennzeichnet, dass** Oligonucleotid-Primer der Sequenz SEQ ID NO:2 oder SEQ ID NO:3 als Gensonde dienen und mit Acarbose-Biosynthesegenen hybridisieren.

7. Verfahren zur Herstellung von Acarbose, **dadurch gekennzeichnet, daß** man Mikroorganismen gemäß Anspruch 4 kultiviert und die Acarbose aus der Kulturbrühe isoliert.

8. Verwendung von DNA Molekülen gemäß Anspruch 2 für die Identifizierung weiterer Acarbose-Strukturgene und -Regulationsgene in Actinoplanes.

9. Verwendung von DNA Molekülen gemäß Anspruch 2 zur Verbesserung der Acarbose-Syntheseleistung von Mikroorganismen durch Steigerung der Gendosis besagter DNA Moleküle, oder durch Verwendung effektiverer Promotoren oder durch gentechnische Veränderung der Regulation (z.B. der Regulatorproteinexpression oder der Regulator-Erkennungsstellen).

10. Verwendung von DNA Molekülen gemäß Anspruch 2 zur Isolierung von Biosynthesegenen für Acarbose-verwandte Naturstoffe.

11. Verwendung von DNA Molekülen gemäß Anspruch 2 zur Modifikation von Biosyntheseenzymen mittels Protein Engineering.

## Claims

1. DNA molecule according to SEQ ID 01.

2. Fragments of DNA molecules according to Claim 1, containing
(i) the complete acbB DNA sequence coding for dTDP-glucose dehydratase, and/or
(ii) a partial sequence of the acbA gene, coding for dTDP-glucose synthase with a sequence according to position 2081 to 2223 in SEQ ID NO: 1, and/or
(iii)a partial sequence of the acbC gene, coding for a cyclase with a sequence according to position 1 to 929 in SEQ ID NO: 1.

3. Vectors containing a DNA according to Claims 1 and 2.

4. Microorganisms transformed with the vector according to Claim 3.

5. Polypeptides encoded by DNA molecules according to Claims 1 and 2.

6. Process for the isolation of one or more acarbose biosynthesis genes from actinoplanes, **characterized in that** oligonucleotide primers of the sequence SEQ ID NO: 2 or SEQ ID NO: 3 are used as gene probe and hybridize with acarbose biosynthesis genes.

7. Process for the preparation of acarbose, **characterized in that** microorganisms according to Claim 4 are cultivated, and the acarbose is isolated from the culture broth.

8. Use of DNA molecules according to Claim 2 for identifying further acarbose structural genes and regulatory genes in actinoplanes.

9. Use of the DNA molecules according to Claim 2 for improving the acarbose synthetic output by increasing the gene dose of said DNA molecules, or by using more active promoters or by altering the regulation by genetic manipulation (for example of regulator protein expression or of regulator recognition sites).

10. Use of DNA molecules according to Claim 2 for isolating biosynthesis genes for acarbose-related natural substances.

11. Use of DNA molecules according to Claim 2 for modifying biosynthetic enzymes by protein engineering.

## Revendications

1. Molécule d'ADN selon SEQ ID N° 1.

2. Fragments de molécules d'ADN suivant la revendication 1, contenant
(i) la séquence complète d'ADN de acbB, codant la dTDP-glucose-déshydratase, et/ou
(ii) une séquence partielle du gène acbA, codant la dTDP-glucose-synthase avec une séquence selon les positions 2081 à 2223 dans SEQ ID N° 1, et/ou
(ii) une séquence partielle du gène acbC, codant une cyclase avec une séquence selon la position 1 à 929 dans SEQ ID N° 1.

3. Vecteurs contenant un ADN selon les revendications 1 et 2.

4. Microorganismes transformés avec le vecteur suivant la revendication 3.

5. Polypeptides codés par des molécules d'ADN selon les revendications 1 et 2.

6. Procédé pour isoler des gènes d'Actinoplanes ou plusieurs gènes de biosynthèse de l'acarbose, **caractérisé en ce que** des amorces oligonucléotidiques de la séquence SEQ ID N° 2 ou SEQ ID N° 3 servent de sonde génique et sont hybridées avec des gènes de biosynthèse de l'acarbose.

7. Procédé de production d'acarbose, **caractérisé en ce qu'**on cultive des microorganismes suivant la revendication 4 et on isole l'acarbose du bouillon de culture.

8. Utilisation de molécules d'ADN suivant la revendication 2 pour l'identification d'autres gènes de structure et gènes de régulation de l'acarbose dans Actinoplanes.

9. Utilisation de molécules d'ADN suivant la revendication 2 pour améliorer la capacité de synthèse de l'acarbose de microorganismes par augmentation de la dose géniques des molécules d'ADN en question par l'utilisation de promoteurs plus efficaces ou par modification par une technique génique de la régulation (par exemple de l'expression de la protéine du régulateur ou des sites de reconnaissance du régulateur).

10. Utilisation de molécules d'ADN suivant la revendication 2 pour l'isolement de gènes de biosynthèse pour des substances naturelles apparentées à l'acarbose.

11. Utilisation de molécules d'ADN suivant la revendication 2 pour la modification d'enzymes de biosynthèse au moyen de la technique de modification génétique des protéines.


### SEQUENZPROTOKOLL

(1) ALLGEMEINE ANGABEN:
   (i) ANMELDER:
      (A) NAME: Bayer AG
      (B) STRASSE: Bayerwerk
      (C) ORT: Leverkusen
      (E) LAND: Deutschland
      (F) POSTLEITZAHL: 51368
      (G) TELEFON: 0214-3061455
      (H) TELEFAX: 0214-303482
   (ii) BEZEICHNUNG DER ERFINDUNG: Acarbose Gene
   (iii) ANZAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 2219 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Actinoplanes sp. SE 50/110
   (xi) SEQUENZBESCHREIBUNG: SEQ ID NO: 1:
   GGATCCGGGA GTACTTCACC CATCACGGCA TCGATCATTC GATCCTGGTG ATGCGGGTGG     60
   GCGAGACGGT CAAGGACTTC GACACGGCGG GCCGCATCGT CGCCGCGATG GACGCCTTCG    120
   GACTGGCCCG CCGCCGGGAG CCGATGATCG TCGTCGGTGG TGGGGTGCTG ATGGACGTGG    180
   CCGGTCTGGT GGCCAGCCTC TACCGGGCGC GGCACGCCGT TCTGCGGGTG CCGACGACAC    240
   TGGTCGGACT GATCGACGCG GTGTCGCGCG AAGACCGGGT CAACTTCAAC GGCCACAAGG    300
   AACCGGCTGG GTACGTACGC CCGGCTGATC TGACCCTGCT GGACCGCCGC TTCCTGGCCA 360
   CCCTGGACCG GCGCCACCTC AGCAACGGGC TCGCCGAGAT GCTCAAGATC GCGCTGATCA 420
   AGGATGCCGA GCTGTTCCAG CTGCTGGAGC GGCACGGGCG GGTCCTGATC GAGGAACGGT 480
   TCCAGGGCGT ACCGGAACCG GTGACCGGGC CGCCGTCCGG GCCCTGCGCG CGCCACCCAT 540
   GGCATGCTGG AGGAACTCGG CCCAATCTGT GGGAGAGCCG GCTGGAACGC AGTGTCGACT 600
   ACGGGCACAC GTTCAGCCCG ACCATCGAGA TGCGCGCGCT GCCGGCTCTG CTGCACGGCG 660
   AGGCCGTGTG TGTGGACATG GCGCTGACCA CGGTGCTGGC GTACCGGCGG GGTCTGCTCG 720
   ACGTCGCGCA GCGGGACCGG ATCTTCGCGG TGATGACCGC CCTGGGCCTG CCGACCGGCA 780
   TCCGCCTGCT CACGCCGGAG GTGCTGGAGG CGGCGTTGCA GGACACCGTC CGGCACCGGG 840
   ACGGGTGGCA GCGGCTGCCA CTGCCGGTGG GGATCGGGGG TGTCACGTTC GTCAACGACG 900
   TGACGGCCGC CGAGCTGCAG CCGCCGCGCT GATGCAGCAC CGGCTCGCCG AGGACGCCCT 960
   GCTGCTGCGC GCCCTAGCTC GGGCCGCGGA CGCCGATCGC CGGAAGCGAC CGGCGTCCGT 1020
   CCGCCCACCG GTTGCCGTCA GTCCACCAGG AACGGTTGGC GCGATACCAC GCGACCGTTT 1080
   CCGCGATGCC GTCGGTGAAA TCGACCCGCG GCCGGTAACC GAGTTCCCCG GCGATTTTCG 1140
   AATAGTCGAG AGAATAGCGC CGGTCGTGAC CTTTGCGATC GGTCACGAAA GATATGCGCG 1200
   AACGCCGGGC GCCGCACGCC TCGAGGAGGA TCTCGGTCAA TTCGAGATTC GTCGCCTCCC 1260
   ACCCACCGCC GATGTGATAG ACCTCGCCTG CCCGGCCGGC ACCCAGGGCC AGGGCGAGAC 1320
   CGCGGCAATG GTCGCTGACG TGGAGCCAGT CGCGGATGTT GCGGCCGTCG CCGTAGACCG 1380
   GTACGTCGAG CCCGTCGAGC AGCCTGGTGA CGAACAGCGG AATCATTTTC TCCGGGAATT 1440
   GCCGGGGCCC GTAGTTGTTG GAGCAGCGGG TCACCACGAC GTCCATCCCG TGCGTCTGGT 1500
   GGTAGGCCAG AGCGAGGAGG TCGGACCCGG CTTTGCTCGC GGCGTACGGC GAGTTGGGCG 1560
   CCAGCGGATG GCCCTCGGCC CACGAGCCGG TGTCGATCGA CCCGTACACC TCGTCGGTGG 1620
   AAACATGCAG GAAGCGGCCG ATATGGTGGC GTAGCGCGGC GTCCAGTAGC ACCTGAGTGC 1680
   CGACCAGGTT GCTGGCCACG AAGGGGCCGG AGGCGACCAC CGAGCGGTCG ACGTGGGTCT 1740
   CGGCGGCGAA GTGCGCCACG GTGTCGTGCC GCGCCATCAG CCCCTCGATT AGACCTTCGT 1800
   CACAGATGTC GCCCCGAACG AAGCTGAAAC GAGGGTCCGC CGACGCTTCG GCGAGATTTC 1860
   TGAGATTGCC TCCGTAACCC AGTTTGTCGA CGACCGTAAC CTGCGTCACG GGTTGTGGTG 1920
   TGGCAATGTC GCCACTGATC AGGGAAGTTA CAAAATGGGA CCCGATAAAG CCGGCTCCGC 1980
   CGGTGACCAA GATTTTCATC GCCGGGATTG TAGCAATGCC GCCAATGGGT GCCCGATGTT 2040
   CGGCCGAGCC ATTTACGGGG CTTGCTGATA TGGTCGGTCA CGTGCGCGGA ATATTGCTGG 2100
   CCGGGGGAAC CGGCTCACGG CTTCGACCGG TGACCTGGGC GGTTTCCAAA CAACTGATGC 2160
   CGGTCTATGA CAAACCGATG ATCTACTATC CGCTGGCCAC GCTCGTCAGC TGCGGATCC 2219

### SEQUENCE LISTING

<110> Bayer AG
<120> Acarbose Biosynthesegene aus Actinoplanes
<130> LeA30515-EP
<140> EP0730029
   <141> 1996-02-17
   <150> DE19507214
   <151> 1995-03-02
<160> 3
   <170> PatentIn version 3.1
<210> 1
   <211> 2219
   <212> DNA
   <213> Actinoplanes
   <400> 1
   ggatccggga gtacttcacc catcacggca tcgatcattc gatcctggtg atgcgggtgg     60
   gcgagacggt caaggacttc gacacggcgg gccgcatcgt cgccgcgatg gacgccttcg    120
   gactggcccg ccgccgggag ccgatgatcg tcgtcggtgg tggggtgctg atggacgtgg    180
   ccggtctggt ggccagcctc taccgggcgc ggcacgccgt tctgcgggtg ccgacgacac    240
   tggtcggact gatcgacgcg gtgtcgcgcg aagaccgggt caacttcaac ggccacaagg    300
   aaccggctgg gtacgtacgc ccggctgatc tgaccctgct ggaccgccgc ttcctggcca    360
   ccctggaccg gcgccacctc agcaacgggc tcgccgagat gctcaagatc gcgctgatca    420
   aggatgccga gctgttccag ctgctggagc ggcacgggcg ggtcctgatc gaggaacggt    480
   tccagggcgt accggaaccg gtgaccgggc cgccgtccgg gccctgcgcg cgccacccat    540
   ggcatgctgg aggaactcgg cccaatctgt gggagagccg gctggaacgc agtgtcgact    600
   acgggcacac gttcagcccg accatcgaga tgcgcgcgct gccggctctg ctgcacggcg    660
   aggccgtgtg tgtggacatg gcgctgacca cggtgctggc gtaccggcgg ggtctgctcg    720
   acgtcgcgca gcgggaccgg atcttcgcgg tgatgaccgc cctgggcctg ccgaccggca    780
   tccgcctgct cacgccggag gtgctggagg cggcgttgca ggacaccgtc cggcaccggg    840
   acgggtggca gcggctgcca ctgccggtgg ggatcggggg tgtcacgttc gtcaacgacg    900
   tgacggccgc cgagctgcag ccgccgcgct gatgcagcac cggctcgccg aggacgccct    960
   gctgctgcgc gccctagctc gggccgcgga cgccgatcgc cggaagcgac cggcgtccgt   1020
   ccgcccaccg gttgccgtca gtccaccagg aacggttggc gcgataccac gcgaccgttt   1080
   ccgcgatgcc gtcggtgaaa tcgacccgcg gccggtaacc gagttccccg gcgattttcg   1140
   aatagtcgag agaatagcgc cggtcgtgac ctttgcgatc ggtcacgaaa gatatgcgcg   1200
   aacgccgggc gccgcacgcc tcgaggagga tctcggtcaa ttcgagattc gtcgcctccc   1260
   acccaccgcc gatgtgatag acctcgcctg cccggccggc acccagggcc agggcgagac   1320
   cgcggcaatg gtcgctgacg tggagccagt cgcggatgtt gcggccgtcg ccgtagaccg   1380
   gtacgtcgag cccgtcgagc agcctggtga cgaacagcgg aatcattttc tccgggaatt   1440
   gccggggccc gtagttgttg gagcagcggg tcaccacgac gtccatcccg tgcgtctggt   1500
   ggtaggccag agcgaggagg tcggacccgg ctttgctcgc ggcgtacggc gagttgggcg   1560
   ccagcggatg gccctcggcc cacgagccgg tgtcgatcga cccgtacacc tcgtcggtgg   1620
   aaacatgcag gaagcggccg atatggtggc gtagcgcggc gtccagtagc acctgagtgc   1680
   cgaccaggtt gctggccacg aaggggccgg aggcgaccac cgagcggtcg acgtgggtct   1740
   cggcggcgaa gtgcgccacg gtgtcgtgcc gcgccatcag cccctcgatt agaccttcgt   1800
   cacagatgtc gccccgaacg aagctgaaac gagggtccgc cgacgcttcg gcgagatttc   1860
   tgagattgcc tccgtaaccc agtttgtcga cgaccgtaac ctgcgtcacg ggttgtggtg   1920
   tggcaatgtc gccactgatc agggaagtta caaaatggga cccgataaag ccggctccgc   1980
   cggtgaccaa gattttcatc gccgggattg tagcaatgcc gccaatgggt gcccgatgtt   2040
   cggccgagcc atttacgggg cttgctgata tggtcggtca cgtgcgcgga atattgctgg   2100
   ccgggggaac cggctcacgg cttcgaccgg tgacctgggc ggtttccaaa caactgatgc   2160
   cggtctatga caaaccgatg atctactatc cgctggccac gctcgtcagc tgcggatcc    2219
<210> 2
   <211> 21
   <212> DNA
   <213> Artificial Sequence
   <220>
   <223> Primer AS2
   <400> 2
   gccgccgaat cccatgtgga c                                                21
<210> 3
   <211> 24
   <212> DNA
   <213> Artificial Sequence
   <220>
   <223> Primer AS5
   <400> 3
   cccgtagttg ttggagcagc gggt                                            24